**Europäisches Patentamt**

**European Patent Office**

**Office européen des brevets**

(19)

(11) Veröffentlichungsnummer: **0 146 882**
**B1**

# EUROPÄISCHE PATENTSCHRIFT

(12)

(45) Veröffentlichungstag der Patentschrift:
22.07.87

(21) Anmeldenummer: 84115284.6

(22) Anmeldetag: 12.12.84

(51) Int. Cl.⁴: **C 07 C 19/045,** C 07 C 17/02, C 07 C 17/38

(54) Verfahren zur Herstellung von 1,2-Dichlorethan.

(30) Priorität: 27.12.83 DE 3347153

(43) Veröffentlichungstag der Anmeldung:
03.07.85 Patentblatt 85/27

(45) Bekanntmachung des Hinweises auf die Patenterteilung:
22.07.87 Patentblatt 87/30

(84) Benannte Vertragsstaaten:
BE CH DE FR GB IT LI NL SE

(56) Entgegenhaltungen:
DE-A-3 148 450
DE-A-3 247 988
DE-B-1 768 367

(73) Patentinhaber: HOECHST AKTIENGESELLSCHAFT,
Postfach 80 03 20, D-6230 Frankfurt am Main 80
(DE)

(72) Erfinder: Hundeck, Joachim, Dr., Argelander
Strasse 135, D-5300 Bonn (DE)
Erfinder: Kühn, Wenzel, Dr., Kampenwandstrasse
15, D-8269 Burgkirchen/Alz (DE)
Erfinder: Scholz, Harald, Dr., Am Beissel 8, D-5042
Erftstadt (DE)
Erfinder: Schaffelhofer, Iwo, Dr., Kettelerstrasse
2, D-8263 Burghausen (DE)

EP 0 146 882 B1

## Beschreibung

Die Herstellung von 1,2-Dichlorethan durch Umsetzung von Ethylen mit Chlor in 1,2-Dichlorethan als Lösemittel und Reaktionsmedium ist bekannt. Zur Beschleunigung der Chloraddition verwendet man als Katalysator neben den Chloriden der Elemente der IV. bis VI. Gruppe des Periodensystems vor allem wasserfreies Eisen-III-chlorid, gegebenenfalls in Gegenwart von Sauerstoff zur Verminderung der Bildung von Nebenprodukten, da Eisen-III-chlorid leicht zugänglich und preisgünstig ist. Als hauptsächliches Nebenprodukt fällt bei dieser Reaktion 1,1,2-Trichlorethan durch Substitution von Dichlorethan an.

Die Chloraddition an Ethylen wird in der Technik sowohl bei Reaktionstemperaturen um 90° C unter Normaldruck, wobei das Reaktionsprodukt 1,2-Dichlorethan (Sdp. 83,7° C) unter Ausnutzung der freigesetzten Reaktionswärme aus dem Reaktionsgemisch abdestilliert wird, als auch bei tieferen Temperaturen von 30-60° C, wie in Ullmanns Encyklopädie der technischen Chemie, Bd. 9, 4. Auflage 1975, Seite 427 beschrieben, durchgeführt. Die Reaktionswärme kann in letzterem Falle nicht für die Rektifikation des Rohdichlorethans herangezogen werden, sondern muß, wie beispielsweise in der DE-AS 19 05 517 ausgeführt, durch Umpumpen des Reaktionsmediums über Wärmetauscher abgeführt werden.

Das bei tieferen Temperaturen hergestellte rohe, katalysatorhaltige Dichlorethan wurde bisher im allgemeinen aus dem Reaktionsgefäß abgezogen und zur Entfernung des Katalysators und des im Rohprodukt enthaltenen Chlorwasserstoffes mit Wasser bzw. wäßrigen Alkalilösungen gewaschen, von der wäßrigen Schicht abgetrennt und anschließend in bekannter Weise destillativ aufgearbeitet.

Die Verwendung von $FeCl_3$ als Katalysator bei der Additionschlorierung von Ethylen ist mit gewissen Nachteilen verbunden. So wirkt $FeCl_3$ in Gegenwart von Wasser gegenüber den metallischen Werkstoffen von Reaktoren, Kolonnen oder Wärmeaustauschern, soweit diese mit dem Katalysator in Berührung kommen, korrosiv. Das zur Chlorierung normalerweise verwendete Chlor mit technischem Reinheitsgrad enthält stets Spuren von Feuchtigkeit, außerdem entsteht immmer Chlorwasserstoff aus unerwünschten Nebenreaktionen.

Nach dem Verfahren der DE-OS 31 48 450 kann die durch $FeCl_3$ als Katalysator bei der Herstellung von 1,2-Dichlorethan verursachte Korrosion in nicht korrosionsfesten Reaktoren erheblich vermindert werden, wenn man den $FeCl_3$-Katalysator mit bestimmten Zusätzen beaufschlagt. Darüberhinaus wurde festgestellt, daß diese Zusätze sich auch vorteilhaft auf die Nebenproduktbildung, welche dadurch verringert wird, auswirken.

Gegenstand der DE-OS 31 48 450 ist ein Katalysatorgemisch, bestehend aus wasserfreiem Eisen-III-chlorid sowie einer weiteren Gemischkomponente zur Herstellung von 1,2-Dichlorethan durch Umsetzung von Ethylen mit Chlor in einem Lösemittel bei Normal- oder Überdruck, welches dadurch gekennzeichnet ist, daß die weitere Gemischkomponente eine in Bezug auf die Eisen-III-chlorid-Menge etwa äquivalente Menge einer Stickstoffbase oder ein Salz dieser Base ist.

Da die beim Verfahren der DE-OS 31 48 450 eingesetzten katalysatorbildenden Komponenten in Form von wasserfreiem $FeCl_3$ und einer Stickstoffbase im Chlorierungsansatz zu einem auch in geringen Mengen hochwirksamen Katalysator reagieren, hat es sich als vorteilhaft erwiesen, diesen Katalysator bei der Aufarbeitung des Chlorierungsgemisches zurückzugewinnen, um ihn erneut für die Umsetzung des Ethylens mit Chlor verwenden zu können. Geeignete Wege zur Aufarbeitung des den Katalysator enthaltenden Chlorierungsgemisches vermittelt die nicht vorveröffentlichte DE-Patentanmeldung P 32 47 988.3.

Gegenstand der DE-Patentanmeldung P 32 47 988.3 ist u.a. ein Verfahren zur Herstellung von 1,2-Dichlorethan durch Umsetzung von Ethylen mit Chlor in einem Lösemittel, vorzugsweise 1,2-Dichlorethan selbst, in Gegenwart eines Katalysatorgemisches bestehend aus wasserfreiem Eisen-III-chlorid und einer Stickstoffbase oder einem Salz dieser Base sowie gegebenenfalls eines Inhibitors zur Verminderung der Nebenproduktbildung bei einer Temperatur unterhalb des Siedepunktes des 1,2-Dichlorethans von etwa 20-100° C und Normal- oder Überdruck und Abtrennen des 1,2-Dichlorethans aus dem Chlorierungsgemisch, welches dadurch gekennzeichnet ist, daß man das Chlorierungsgemisch in eine Destillationskolonne fördert, aus dem Gemisch das 1,2-Dichlorethan bis zur Ausscheidung des Katalysators aus dem flüssigen Sumpfprodukt abdestilliert, aus dem Sumpfprodukt den ausgeschiedenen Katalysator abtrennt und letzteren erneut zur Umsetzung von Ethylen mit Chlor verwendet.

Nunmehr wurde gefunden, daß sich beim Verfahren der DE-Patentanmeldung P 32 47 988.3 das nach Einengen des Reaktionsgemisches der Chlorierung von Ethylen im Sumpf einer Destillationskolonne und anschließender Rückführung eines Katalysatorgemisches aus $FeCl_3$ und z.B. $NH_3$ als Stickstoffbase zunächst gebildete Kristallisat der katalytisch wirksamen Komponenten mit jedem Rückführungsschritt vermindert. Andererseits ergab sich überraschenderweise, daß der flüssige Anteil des Kolonnensumpfes bei der Rückführung in die Reaktionszone gleichfalls volle katalytische Aktivität für die Chlorierung von Ethylen besaß.

Gegenstand vorliegender Erfindung ist somit ein Verfahren zur Herstellung von 1,2-Dichlorethan durch Umsetzung von Ethylen mit Chlor in einem Lösemittel bei Temperaturen von 20 - 100° C und Drücken von 1-15 bar, jedoch unterhalb des jeweiligen Siedepunktes von 1,2-Dichlorethan, in Gegenwart eines Katalysatorgemisches bestehend aus äquivalenten Mengen wasserfreien Eisen-III-chlorids und einer Stickstoffbase oder eines Salzes dieser Base sowie gegebenenfalls in Gegenwart eines Inhibitors zur Verminderung der Bildung von Nebenprodukten und Abtrennen des 1,2-Dichlorethans aus dem Chlorierungsgemisch durch Destillation, welches dadurch gekennzeichnet ist, daß man das Chlorierungsgemisch in eine Destillationskolonne fördert, das 1,2-Dichlorethan bis zur beginnenden Ausscheidung des Katalysators abdestilliert und einen solchen Teil des verbleibenden flüssigen Sumpfprodukts zur erneuten Umsetzung von Ethylen mit Chlor in die Reaktionszone zurückführt, daß die Konzentration des Eisen-III-chlorids im Reaktionsansatz 0,001 bis 0,5 Gew%, bezogen auf die Menge des Lösemittels, beträgt.

Darüberhinaus kann das Verfahren der Erfindung wahlweise und bevorzugt dadurch gekennzeichnet sein, daß

a) das Lösemittel 1,2-Dichlorethan ist;

b) die Reaktionszone Füllkörper aus Stahl enthält.

Die in den Chlorierungsreaktor ggf. eingebrachten Stahl-Füllkörper führen unter den Reaktionsbedingungen zur Bildung einer geringen Menge Eisen-III-chlorid. Andererseits können gewisse Katalysatorverluste auch durch direkte Zufuhr von $FeCl_3$ zum Chlorierungsreaktor ausgeglichen werden. In jedem Falle wird gleichzeitig eine etwa äquivalente Menge Ammoniak als Stickstoffbase eingeleitet. Anstelle von Ammoniak kann ein primäres, sekundäres oder tertiäres Alkyl-, Aralkyl-, Aryl- oder alicyclisches Amin oder ein Polyamin als Stickstoffbase dienen. Als Salze dieser Basen können bevorzugt Halogensalze, z.B. Ammoniumchlorid, eingesetzt werden. Die für den Reaktionsablauf optimale Konzentration an Eisen-III-chlorid wird jetzt erreicht, indem zusätzlich ein Teil des Sumpfes der nachgeschalteten Destillationskolonne in den Chlorierungsreaktor (Reaktionszone) zurückgeführt wird. Die jeweils durch Chlorierung eingebrachter Stahlkörper neugebildete Eisenchloridmenge wird im Restsumpf der Destillation zusammen mit den hochsiedenden Nebenprodukten der Chlorierungsreaktion abgeschieden und ausgeschleust.

Ferner kann der Reaktor nach vollständiger Entleerung mit Hilfe des Katalysatorkonzentrates im Sumpf der Destillationskolonne wieder in Betrieb genommen werden, ohne daß frisches Eisen-III-chlorid zugesetzt werden muß.

Die erfindungsgemäße teilweise Katalysatorrückführung ist möglich, weil der flüssige Sumpf der Destillationskolonne bei seiner Rückführung in die Reaktionszone überraschenderweise volle katalytische Aktivität für die Chlorierung von Ethylen behält. Die Rückführung des katalytisch aktiven Sumpfprodukts ermöglicht eine Einsparung von Eisen-III-chlorid, wobei die Katalysatorausbeute bei der Herstellung von 1,2-Dichlorethan um so stärker ansteigt, je mehr Sumpfprodukt zurückgeführt wird. Gleichzeitig ermöglicht die Sumpfrückführung eine Einsparung an Stickstoffbase, weil der rückgeführte Katalysator bereits die erforderliche Menge an z.B. Ammoniak bzw. Ammonchlorid enthält. Schließlich wird die Abwasserbelastung vermindert, da nur eine geringere Katalysatormenge entsorgt werden muß.

Das Verfahren der Erfindung kann beispielsweise in dem in der DE-A-24 27 045 beschriebenen Schlaufenreaktor durchgeführt werden.

**Beispiel**

Ein zylindrischer Reaktor, dessen Volumen 25 $m^3$ betrug, wurde mit 20 000 Litern 1,2-Dichlorethan beschickt. Der Reaktor enthielt im unteren Teil eine Füllung von Raschig-Ringen aus Stahl. In der Reaktionsflüssigkeit war ein Katalysatorgemisch aus Eisen-III-chlorid und einer dazu äquivalenten Menge Ammoniak enthalten, dessen $FeCl_3$-Gehalt zu 137 mg/kg bestimmt worden war. Bei einem Druck von 1,3 bar am Kopf des Reaktors und einer Temperatur des Reaktionsgemisches von 40° C wurden in den Reaktor stündlich 2326 kg Ethylen und 5884 kg Chlorgas, welches zusätzlich etwa 3,5 Vol% inerte Gase enthielt, sowie 20 $m^3$ Luft als Inhibitor zur Verminderung der Nebenproduktbildung und 40 l $NH_3$ eingeleitet.

Das Reaktionsgemisch wurde in eine Destillationskolonne gefördert, in der 8173 kg/h 1,2-Dichlorethan mit einer Reinheit von 99,9 % abgetrennt werden konnten. 70 % des Kolonnensumpfes wurden in die Reaktionszone zurückgeführt, die restliche Menge wurde in bekannter Weise aufgearbeitet und der darin enthaltene Katalysatoranteil zusammen mit den hochsiedenden Nebenprodukten einer Verbrennungsanlage zugeführt.

Die Bilanz über 96 Stunden ergab eine Ausbeute von 99,6 % 1,2-Dichlorethan, bezogen auf das eingesetzte Ethylen.

**Patentansprüche**

1. Verfahren zur Herstellung von 1,2-Dichlorethan durch Umsetzung von Ethylen mit Chlor in einem Lösemittel bei Temperaturen von 20 - 100° C und Drücken von 1 - 15 bar, jedoch unterhalb des jeweiligen Siedepunktes von 1,2-Dichlorethan, in Gegenwart eines Katalysatorgemisches bestehend aus äquivalenten Mengen wasserfreien Eisen-III-chlorids und einer Stickstoffbase oder eines Salzes dieser Base sowie gegebenenfalls in Gegenwart eines Inhibitors zur Verminderung der Bildung von Nebenprodukten und Abtrennen des 1,2-Dichlorethans aus dem Chlorierungsgemisch durch Destillation, dadurch gekennzeichnet, daß man das Chlorierungsgemisch in eine Destillationskolonne fördert, das 1,2-Dichlorethan bis zur beginnenden Ausscheidung des Katalysators abdestilliert und einen solchen Teil des verbleibenden flüssigen Sumpfprodukts zur erneuten Umsetzung von Ethylen mit Chlor in die Reaktionszone zurückführt, daß die Konzentration des Eisen-III-chlorids im Reaktionsansatz 0,001 bis 0,5 Gew%, bezogen auf die Menge des Lösemittels, beträgt.

2. Verfahren nach Anspruch 1, dadurch gekennzeichnet, daß das Lösemittel 1,2-Dichlorethan ist.

3. Verfahren nach einem der Ansprüche 1 oder 2, dadurch gekennzeichnet, daß die Reaktionszone Füllkörper aus Stahl enthält.

## Claims

1. Process for making 1,2-dichloroethane by reacting ethylene with chlorine in a solvent at temperatures of 20-100°C and under pressures of I-15 bars, but lower than the respective boiling point of 1,2-dichloroethane, in the presence of a catalyst mixture consisting of equivalent quantities of anhydrous iron(III)chloride and a nitrogen base or a salt of said base and, optionally in the presence of an inhibitor reducing the formation of byproducts, and distillatively separating the 1,2-dichloro-ethane from the chlorination mixture, which comprises: introducing the chlorination mixture into a distilling column, distilling off the 1,2-dichloroethane until the catalyst commences depositing, and recycling into the reaction zone for further reaction of ethylene with chlorine, a portion of the remaining liquid still product such that the concentration of the iron(III)chloride in the reaction batch is 0.001 - 0.5 wgt %, based on the quantity of solvent.

2. Process as claimed in claim 1, wherein the solvent is 1,2-dichloroethane.

3. Process as claimed in claim 1 or 2, wherein the reaction zone contains steel packings.

## Revendications

1. Procédé de préparation de 1,2-dichloroéthane par réaction d'éthylène avec du chlore dans un solvant à des températures de 20-100°C, et sous des pressions de 1 - 15 bars, mais au-dessous du point d'ébullition respectif du 1,2-dichloroéthane en présence d'un mélange catalytique constitué par des quantités équivalenres de chlorure de fer (III) anhydre et d'une base azotée ou d'un sel de cette base, et éventuellement en présence d'un inhibiteur pour réduire la formation de produits secondaires, et séparation du 1,2-dichloroéthane du mélange de chloration par distillation, caractérisé en ce que l'on envoie le mélange de chloration dans une colonne de distillation, on sépare le 1,2-dichloroéthane par distillation jusqu'à ce que le catalyseur commence à se déposer et on recycle dans la zone de réaction pour une nouvelle réaction de l'éthylène avec le chlore une partie du résidu liquide de distillation telle que la concentration du chlorure de fer (III) dans la charge réactionnelle soit de 0,001 - 0,5 % en poids, par rapport à la quantité de solvant.

2 Procédé selon la revendication 1, caractérisé en ce que le solvant est le 1,2-dichloroéthane.

3. Procédé selon la revendication 1 ou 2, caractérisé en ce que la zone de réaction contient des corps de remplissage en acier.